# EUROPEAN PATENT APPLICATION

(11) **EP 4 647 077 A1**
(43) Date of publication of application: **12.11.2025**
(21) Application number: 24861797.9
(22) Date of filing: 15.08.2024
(51) Int. Cl.: A61K 31/58, A61K 31/506, A61K 9/00, A61K 9/12, A61P 17/14

(54) **PHARMACEUTICAL FORMULATION FOR TREATING ALOPECIA, PREPARATION METHOD THEREFOR AND USE THEREOF**

(30) Priority: 06.09.2023 CN 202311147688; 06.02.2024 CN 202410167661
(71) Applicant: Beijing Dayspring Pharmaceutical Technology Co., Ltd., Beijing 102206 (CN)
(72) Inventor: YU, Baohui, Beijing 102206 (CN)
(74) Representative: Dompatent Partnerschaft von Patent- und Rechtsanwälten mbB
(86) International application number: PCT/CN2024/112319
(87) International publication number: WO 2025/050953

(57) **Abstract**

The present invention provides a pharmaceutical formulation for treating alopecia, a preparation method therefor and the use thereof. Specifically, the present invention provides a formulation or composition for treating alopecia, comprising the following active components on the basis of the mass percentage (w/w) or mass/volume percentage (w/v): (a) 5% of minoxidil; and (b) 0.07-0.095% of finasteride; the formulation or the composition is a foaming agent or a liniment; and when the formulation is a foaming agent, the total mass in the mass percentage does not contain the mass of a propellant. The foaming agent and liniment of the present invention take effect quickly and have good effects and few side effects.

## Description

### TECHNICAL FIELD

The present invention belongs to the field of pharmaceutical preparations, and specifically, it relates to pharmaceutical formulation for treating alopecia, preparation method therefor and use thereof.

### BACKGROUND

Currently, alopecia is increasingly becoming a problem for people of all ages. Alopecia not only affects the patient's appearance, but also has a significant negative impact on their mental health and social activity.

Taking androgenic alopecia (AGA) as an example, it is the most common type of alopecia and a progressive alopecia characterized by hair follicle miniaturization that begins from adolescence or late adolescence. Both male and female can suffer but appear to a different pattern of alopecia and prevalence. Although AGA does not affect physical health, it seriously affects the mental health and life quality of patients. Early diagnosis and treatment can significantly delay the progression of hair loss and improve the life quality of patients.

Since AGA is a progressive process leading to baldness, the importance of early and long-term treatment should be emphasized. Generally, the earlier the treatment, the better the therapeutic effect. The treatment methods include systemic medication, topical medication, hair transplantation, mesodermal therapy, low-energy laser therapy and so on.

However, the main drugs currently used for treating alopecia still have many shortcomings, such as slow onset, unsatisfactory effects, or side effects.

Therefore, it is greatly important in this field to develop a pharmaceutical composition or a compound formulation for alopecia treatment that takes effect quickly and has good effects and few side effects.

### SUMMARY OF THE INVENTION

The present invention provides a pharmaceutical composition for alopecia treatment that takes effect quickly and has good effects and few side effects.

In the first aspect of the present invention, it provides a formulation or composition for treating alopecia, comprising the following active components because of the mass percentage (w/w) or mass/volume percentage (w/v):
(a) 5% minoxidil; and
(b) 0.07-0.095% finasteride.

In another preferred embodiment, the formulation or composition comprises the following active components on the basis of the mass percentage (w/w) or mass/volume percentage (w/v):
(a) 5% minoxidil; and
(b) 0.07-0.09% finasteride.

In another preferred embodiment, the formulation or composition comprises the following active components on the basis of the mass percentage (w/w) or mass/volume percentage (w/v):
(a) 5% minoxidil; and
(b) 0.07-0.085% finasteride.

In another preferred embodiment, the formulation or composition comprises the following active components on the basis of the mass percentage (w/w) or mass/volume percentage (w/v):
(a) 5% minoxidil; and
(b) 0.07-0.08% finasteride.

In another preferred embodiment, the formulation or composition comprises the following active components on the basis of the mass percentage (w/w) or mass/volume percentage (w/v):
(a) 5% minoxidil; and
(b) 0.07-0.075% finasteride.

In another preferred embodiment, the formulation or composition comprises the following active components on the basis of the mass percentage (w/w) or mass/volume percentage (w/v):
(a) 5% minoxidil; and
(b) 0.075% finasteride.

In another preferred embodiment, the formulation or composition further comprises a pharmaceutically acceptable carrier.

In another preferred embodiment, the dosage form of the formulation or composition is selected from the group consisting of a foam formulation, a liniment formulation, a tincture, a spray, and a gel.

In another preferred embodiment, the dosage form of the formulation or composition is a foam formulation or a liniment formulation.

In another preferred embodiment, the formulation or composition is a foam formulation.

In another preferred embodiment, the formulation or composition is a liniment formulation.

In another preferred embodiment, the formulation or composition may or may not comprise propylene glycol and isopropanol.

In another preferred embodiment, when the formulation or composition is a foam formulation, the formulation or composition does not comprise propylene glycol or isopropanol.

In another preferred embodiment, when the formulation or composition is a foam formulation, the active component is calculated as a mass percentage (w/w).

In another preferred embodiment, when the formulation or composition is a liniment formulation, the active component is calculated as a mass-volume percentage (w/v).

In another preferred embodiment, the formulation is a laboratory formulation.

In another preferred embodiment, the formulation is a pharmaceutical formulation.

In another preferred embodiment, the composition is a pharmaceutical composition.

In another preferred embodiment, the formulation or composition further comprises an additional component that promotes hair growth.

In the second aspect of the invention, it provides a foam formulation for treating alopecia, comprising the following active components on the basis of the mass percentage (w/w):
(a) 5% minoxidil; and
(b) 0.07-0.095% finasteride.

In another preferred embodiment, the foam formulation comprises the following active components on the basis of the mass percentage:
(a) 5% minoxidil; and
(b) 0.07-0.09% finasteride.

In another preferred embodiment, the foam formulation comprises the following active components on the basis of the mass percentage:
(a) 5% minoxidil; and
(b) 0.07-0.085% finasteride.

In another preferred embodiment, the foam formulation comprises the following active components on the basis of the mass percentage:
(a) 5% minoxidil; and
(b) 0.07-0.08% finasteride.

In another preferred embodiment, the foam formulation comprises the following active components on the basis of the mass percentage:
(a) 5% minoxidil; and
(b) 0.07-0.075% finasteride.

In another preferred embodiment, the foam formulation comprises the following active components on the basis of the mass percentage:
(a) 5% minoxidil; and
(b) 0.075% finasteride;
wherein the total mass in the mass percentage does not comprise the mass of the propellant.

In another preferred embodiment, the mass percentage is the percentage of the mass of the active component in the total mass of the foam formulation, and the total mass of the foam formulation does not comprise the mass of the propellant.

In another preferred embodiment, the foam formulation further comprises a pharmaceutically acceptable carrier.

In another preferred embodiment, the foam formulation further comprises a component selected from the group consisting of a humectant, solubilizer, a pH regulator, an antioxidant, a foam stabilizer, diluent, a **chelating agent,** a propellant, and combinations thereof.

In another preferred embodiment, the humectant is selected from the group consisting of glycerol, polyethylene glycol 2000, polyethylene glycol 4000, polyethylene glycol 6000, and combinations thereof.

In another preferred embodiment, the humectant is glycerol.

In another preferred embodiment, the mass percentage of the humectant in the foam formulation is 1-10 wt%; preferably 1-5 wt%; preferably 2-3 wt%; and more preferably 2-2.5 wt%.

In another preferred embodiment, the solubilizer is selected from the group consisting of Tween 40, Tween 60, Tween 80, sodium dodecyl sulfate, docusate sodium, and combinations thereof.

In another preferred embodiment, the solubilizer is Tween 60.

In another preferred embodiment, the mass percentage of the solubilizer in the foam formulation is 0.1-5.0 wt%; preferably 0.1-1.0 wt%; more preferably 0.1-0.5 wt%; and more preferably 0.3-0.5 wt%.

In another preferred embodiment, the pH regulator is selected from the group consisting of lactic acid, hydrochloric acid, sodium hydroxide, acetic acid, and combinations thereof.

In another preferred embodiment, the pH regulator is lactic acid.

In another preferred example, the mass percentage of the pH regulator in the foam formulation is 0.1-5.0 wt%; preferably 0.5-2.5 wt%; more preferably 1.0-2.0 wt%; and more preferably 1.0-1.5 wt%.

In another preferred embodiment, the antioxidant is selected from the group consisting of dibutyl hydroxytoluene (BHT), butyl hydroxyanisole (BHA), vitamin E, and combinations thereof.

In another preferred embodiment, the antioxidant is dibutyl hydroxytoluene.

In another preferred embodiment, the mass percentage of the antioxidant in the foam formulation is 0.01-1 wt%; preferably 0.05-0.5 wt%; and more preferably 0.1-0.2 wt%.

In another preferred embodiment, the foam stabilizer is selected from the group consisting of cetyl alcohol, stearyl alcohol, and combinations thereof.

In another preferred embodiment, the foam stabilizers are cetyl alcohol and stearyl alcohol.

In another preferred example, the mass percentage of the foam stabilizer in the foam agent is 0.5-10 wt%; preferably 0.5-5 wt%; more preferably 1.5-2.5 wt%; and more preferably 1.5-2.0 wt%.

In another preferred embodiment, the diluent is selected from the group consisting of purified water, anhydrous ethanol, n-propanol, and combinations thereof.

In another preferred embodiment, the diluent is selected from the group consisting of purified water, anhydrous ethanol, and combinations thereof.

In another preferred embodiment, the diluent is selected as a mixture of purified water and anhydrous ethanol.

In another preferred embodiment, the chelating agent is selected from the group consisting of lactic acid, anhydrous citric acid, acetic acid, malic acid, maleic acid, and combinations thereof.

In another preferred embodiment, the chelating agent is anhydrous citric acid.

In another preferred example, the mass percentage of the chelating agent in the foam formulation is 0.1-5.0 wt%; preferably 0.1-1.0 wt%; more preferably 0.1-0.5 wt%; more preferably 0.1-0.3 wt%; and more preferably 0.1-0.2 wt%.

In another preferred embodiment, the propellant is selected from the group consisting of propane, butane, isobutane, and combinations thereof.

In another preferred embodiment, the propellant comprises: a mixture of 48% propane, 30% butane, and 22% isobutane, calculated by mass percentage (w/w).

In another preferred embodiment, the mass percentage of the propellant in the foam formulation is 1.5-8.5 wt%; preferably 2.5-7.5 wt%; more preferably 3.5-6.5 wt%; and more preferably 4.5-5.5 wt%.

In another preferred embodiment, the foam formulation does not contain propylene glycol or isopropanol.

In another preferred embodiment, the foam formulation further comprises: glycerol, Tween 60, lactic acid, dibutyl hydroxytoluene, cetyl alcohol, stearyl alcohol, anhydrous citric acid, anhydrous ethanol, purified water and a propellant.

In another preferred embodiment, calculated on a propellant-free basis, the foam formulation comprises 2.11% glycerol, 0.42% Tween 60, 1.05% lactic acid, 0.1% dibutyl hydroxytoluene, 1.16% cetyl alcohol, 0.53% stearyl alcohol, 0.11% anhydrous citric acid, 56.46% anhydrous ethanol, and 32.985% purified water, calculated by mass percent (w/w).

In another preferred embodiment, the foam formulation further comprises: 5.00% of the propellant, calculated by mass percentage (w/w).

In another preferred embodiment, the foam formulation further comprises an additional component to promote hair growth.

In the third aspect of the present invention, it provides a liniment formulation for treating alopecia, comprising the following active components on the basis of the mass/volume percentage (w/v):
(a) 5% minoxidil; and
(b) 0.07-0.095% finasteride.

In another preferred embodiment, the liniment formulation comprises the following active components on the basis of the mass/volume percentage:
(a) 5% minoxidil; and
(b) 0.07-0.09% finasteride.

In another preferred embodiment, the liniment formulation comprises the following active components on the basis of the mass/volume percentage:
(a) 5% minoxidil; and
(b) 0.07-0.085% finasteride.

In another preferred embodiment, the liniment formulation comprises the following active components on the basis of the mass/volume percentage:
(a) 5% minoxidil; and
(b) 0.07-0.08% finasteride.

In another preferred embodiment, the liniment formulation comprises the following active components on the basis of the mass/volume percentage:
(a) 5% minoxidil; and
(b) 0.07-0.075% finasteride.

In another preferred embodiment, the liniment formulation comprises the following active components on the basis of the mass/volume percentage:
(a) 5% minoxidil; and
(b) 0.075% finasteride.

In another preferred embodiment, the mass/volume percentage is the percentage of the active component mass (g) to the total volume of the liniment formulation (ml).

In another preferred embodiment, the liniment formulation further comprises a pharmaceutically acceptable carrier.

In another preferred embodiment, the liniment formulation further comprises a penetration enhancer, a cosolvent, a solvent, or combinations thereof.

In another preferred embodiment, the penetration enhancer is selected from the group consisting of propylene glycol, laurocapram, cyclohexanol, decyl methyl sulfoxide, and combinations thereof.

In another preferred embodiment, the penetration enhancer is propylene glycol.

In another preferred embodiment, the volume percentage (v/v) of the penetration enhancer in the liniment formulation is 35%-60% (v/v), preferably 45% -55% (v/v), and more preferably 50%-55% (v/v).

In another preferred embodiment, the cosolvent is selected from the group consisting of anhydrous ethanol, glycerol, isopropanol, polyethylene glycol, liquid paraffin, and combinations thereof.

In another preferred embodiment, the cosolvent is anhydrous ethanol.

In another preferred embodiment, the volume percentage (v/v) of the cosolvent in the liniment formulation is 15%-45% (v/v), preferably 20%-40% (v/v), and more preferably 25%-35% (v/v).

In another preferred embodiment, the solvent is purified water.

In another preferred embodiment, the liniment formulation further comprises propylene glycol, anhydrous ethanol, and purified water.

In another preferred embodiment, the liniment formulation further comprises 50% (v/v) propylene glycol and 30% (v/v) anhydrous ethanol.

In another preferred embodiment, the liniment formulation further comprises purified water which is brought to the specified volume.

In another preferred embodiment, the liniment formulation further comprises an additional component for promoting hair growth.

In the fourth aspect of the invention, it provides a use of the formulation or composition of the first aspect of the invention, the foam formulation of the second aspect of the invention, or the liniment formulation of the third aspect of the invention for preparing a pharmaceutical composition for treatment of alopecia.

In another preferred embodiment, the treatment comprises promoting hair growth and preventing further hair loss.

In another preferred embodiment, the alopecia comprises: androgenic alopecia, alopecia caused by drugs (such as chemotherapy drugs), alopecia caused by radiation therapy, alopecia caused by age, alopecia caused by autoimmune disease, alopecia caused by hormone, alopecia caused by infection, or combinations thereof.

In another preferred embodiment, the alopecia is male androgenic alopecia.

In another preferred embodiment, the hair loss is male androgenic alopecia or female postmenopausal alopecia.

In the fifth aspect of the invention, it provides a method for preparing the foam formulation of the second aspect of the invention, comprising the following steps:
(s1) taking the following active components as raw materials according to the mass percentage for subsequent use:
   (a) 5% minoxidil; and
   (b) 0.07-0.095% finasteride;
(s2) mixing the active components with a pharmaceutically acceptable carrier, dissolving, filling and sealing; and
(s3) injecting a propellant into a sealed solution to prepare the foam formulation.

In another preferred embodiment, when the foam formulation is prepared and calculated according to the mass percentage, in step (s2), it further comprises the following substeps:
(s2a) preparing an aqueous phase: dissolving a chelating agent in purified water;
(s2b) preparing an alcohol phase: dissolving finasteride in a small amount of anhydrous ethanol, then adding humectant, solubilizer, pH regulator, finasteride solution, antioxidant, and foam stabilizer into anhydrous ethanol for dissolution, and then adding minoxidil, stirring to disperse; and
(s2c) mixing chelating agent aqueous solution with mixture of substep (s2b), stirring until the minoxidil is completely dissolved, then filling and sealing.

In another preferred embodiment, when the foam formulation is prepared and calculated according to the mass percentage, in step (s2), it further comprises the following substeps:
(s2a) preparing an aqueous phase: dissolving anhydrous citric acid in purified water;
(s2b) preparing an alcohol phase: dissolving finasteride in a small amount of anhydrous ethanol, then adding glycerol, Tween 60, lactic acid, finasteride ethanol solution, dibutyl hydroxytoluene, cetyl alcohol, and stearyl alcohol into anhydrous ethanol for dissolution, and then adding minoxidil, stirring to disperse; and
(s2c) mixing the aqueous phase of substep (s2a) with the alcohol phase solution of substep (s2b), stirring until the minoxidil is completely dissolved, then filling and sealing.

In the sixth aspect of the present invention, it provides a method for preparing the liniment formulation according to the third aspect of the present invention, comprising the following steps:
(s1) taking the following active components as raw materials according to the mass/volume percentage for subsequent use;
   (a) 5% minoxidil; and
   (b) 0.07-0.095% finasteride;
(s2) mixing the active components with a pharmaceutically acceptable carrier evenly, adjusting to final volume, and filtering; and
(s3) filling a filtered solution into a container, thereby obtaining the liniment formulation.

In another preferred embodiment, when the liniment formulation is prepared and calculated by the mass/volume percentage, in step (s2), it further comprises the following substeps:
(s2a) dissolving finasteride in a small amount of cosolvent;
(s2b) mixing a penetration enhancer, a cosolvent, and a first portion of solvent evenly, then adding minoxidil and finasteride solution, stirring to dissolve; and
(s2c) adjusting the volum of solution obtained after dissolution with the remaining solvent to final volum and filtering.

In another preferred embodiment, when the liniment formulation is prepared and calculated by the mass/volume percentage, in step (s2), it further comprises the following substeps:
(s2a) dissolving finasteride in a small amount of anhydrous ethanol;
(s2b) mixing the propylene glycol, anhydrous ethanol, and a first portion of purified water evenly, then adding minoxidil and finasteride ethanol solution, stirring to dissolve; and
(s2c) diluting the solution obtained after dissolution with remaining purified water and filtering.

In the seventh aspect of the invention, it provides a method for treating alopecia, comprising: administering a therapeutically effective amount of the formulation or composition of the first aspect of the present invention, the foam formulation of the second aspect of the present invention, or the liniment formulation of the third aspect of the present invention to a subject in need thereof.

In another preferred embodiment, the method is non-diagnostic and non-therapeutic.

It should be understood that within the scope of the present invention, the above technical features of the present invention and the specific technical features described in the following examples can be combined with each other to form new or preferred technical solutions which will not be redundantly repeated one-by-one due to space limitations.

### BRIEF DESCRIPTION OF DRAWINGS

Figures 1-3 show the hair growth score results for mice in each experimental group after treatment with the foam formulation of the present invention.
Figure 4 shows the hair growth score results for mice in each experimental group after treatment with the liniment formulation of the present invention.

### DETAILED DESCRIPTION

After extensive and intensive research, the inventors have unexpectedly discovered that a specific ratio of active components combination of minoxidil and finasteride (e.g., 5% minoxidil and 0.075% finasteride, on the basis of mass percentage or mass/volume percentage) has much excellent therapeutic effects on alopecia as compared to other ratios, and can take effect quickly and has good effects. On this basis, the present invention is completed.

Specifically, the numerous comparative experiments were conducted in the present invention, wherein single-agent formulations (minoxidil or finasteride) and combination formulations with specific ratios were evaluated in mouse model of alopecia. The experimental results show that when the content of minoxidil is 5% and the content of finasteride is 0.075%, the foam formulation and liniment formulation of the present invention have unexpected excellent effects, and the effect of treating alopecia is better than that of 0.1% finasteride. Further, when the content of finasteride is 0.15%, its effect on treating alopecia is also better than that of 0.1% finasteride. That is, there are two peaks of effect when the content of finasteride is about 0.075% and 0.15%, respectively.

### TERM

In order to facilitate a better understanding of this disclosure, certain terms are first defined. As used in this application, unless otherwise specified in this document, each of the following terms shall have the meaning given below. Other definitions are elaborated throughout the application.

The term "about" may refer to a value or composition within an acceptable range of error determined by the skilled in the art, which will depend in part on how the value or composition is measured or determined. For example, as used herein, the expression "about 100" includes all values between 99 and 101 (e.g., 99.1, 99.2, 99.3, 99.4, etc.).

As used herein, the terms "comprising" or "including" can be open, semi closed, or closed. In other words, the term also includes "consisting essentially of" or "consisting of".

As used herein, unless otherwise specified, any concentration range, percentage range, proportion range, or integer range shall be understood to include the value of any integer within the range and, where appropriate, the value of its fraction (such as one tenth and one percent of an integer).

As used herein, the term "and/or" refers to and encompasses any and all possible combinations of one or more related listed items.

As used herein, the terms "specification 5% -0.075%" and "5% -0.075%" have the same meaning, both of which mean that if it is in foam formulation, the mass percentage of minoxidil is 5%, the mass percentage of finasteride is 0.075%; if it is in liniment formulation, the mass/volume percentage of minoxidil is 5% and the mass/volume percentage of finasteride is 0.075%. Other similar expression herein has same meaning, that is, in foam formulation, the first (left) percentage value is the mass percentage of minoxidil, and the second (right) percentage value is the mass percentage of finasteride; and in the liniment formulation, the first percentage value is the mass/volume percentage of minoxidil, and the second percentage value is the mass/volume percentage of finasteride.

The main advantages of the present invention include:
(a) compared to single formulation of minoxidil, single formulation of finasteride, and compound formulation of minoxidil and finasteride with other ratios and dosage forms, the formulation or composition provided by the present invention exhibits fast onset, good efficacy, and few side effects, and has much better clinical application prospects.
(b) when the pharmaceutical preparation of the present invention is a foam formulation, it has the following advantages compared with other dosage forms: it does not contain propylene glycol or isopropanol, thereby reducing irritation and allergy; the foam is evenly dispersed, easy to spread, widely coated, and has obvious local therapeutic effect; foam has small density and soft structure; after the foam bursts, it is easy to enter the cuticle through the hair follicle, and easy to absorb; foam is easy to use, tasteless, non-sticky, and less residue so that patients are generally more willing to accept it, and the drug compliance is high.

The present invention will be further explained in conjunction with specific examples. It should be understood that these examples are only used to illustrate the present invention and not to limit the scope of the present invention. The experimental methods without specific conditions specified in the following examples are usually carried out under conventional conditions or conditions recommended by the manufacturer. Unless otherwise specified, percentages and portions refer to mass percentages and mass portions or volume portions.

### Example 1 Pharmacodynamic Study 1

### 1.1 Experimental reagents and materials

The experimental materials for this example are shown in Table 1.

**Table 1 Experimental Materials**

| Experimental materials | Source |
|---|---|
| C57BL/6J male mice (6-8 week-aged) | SPF (Beijing) Biotechnology Co., Ltd. |
| Testosterone propionate | Shanghai Macklin Biochemical Co., Ltd. |
| Corn oil for injection | Sigma |
| Blank foam formulation | Self-prepared in this example |
| Minoxidil foam formulation (Specification 5%) | Self-prepared in this example |
| Finasteride | Hubei Gedian Humanwell Pharmaceutical Co., Ltd. |
| Minoxidil-finasteride foam formulation (Specification 5% -0.008%) | Self-prepared in this example |
| Minoxidil-finasteride foam formulation (Specification 5% -0.025%) | Self-prepared in this example |
| Minoxidil-finasteride foam formulation (Specification 5% -0.075%) | Self-prepared in this example |

### Preparation of testosterone propionate solution:

80 mg of testosterone propionate was accurately weighed and dissolved in 26.133 mL of corn oil for injection. Vortex-sonication was applied to obtain a homogeneous solution with a concentration of 3 mg/ml.

### Preparation of finasteride suspension:

0.542 mg of finasteride was weighed accurately and 5.420 ml of 0.5% CMC-Na was added. Vortex-sonication was applied to obtain a 0.1 mg/mL homogeneous suspension.

### Preparation of 5% minoxidil foam formulation:

1) The prescription of composition is shown in Table 2.

**Table 2 Prescription of Composition**

| Component | Mass per can (g) | Proportion (wt%) (excluding propellant) |
|---|---|---|
| Minoxidil | 2.850 | 5.00 |
| Anhydrous ethanol | 32.182 | 56.46 |
| Purified water | 18.844 | 33.06 |
| BHT | 0.057 | 0.10 |
| Lactic acid | 0.599 | 1.05 |
| Anhydrous citric acid | 0.063 | 0.11 |
| Glycerol | 1.203 | 2.11 |
| Cetyl alcohol | 0.661 | 1.16 |
| Stearyl alcohol | 0.302 | 0.53 |
| Tween-60 | 0.239 | 0.42 |
| Propellant | 3.000 | N/A |
| Total | 60.000 | 100.00 |

The composition of the propellant prescription and the filling prescription are shown in Tables 3 and 4.

**Table 3 Composition of Propellant Prescription**

| Composition of propellant (mixed hydrocarbons) | Proportion (%) |
|---|---|
| Propane | 48 |
| Butane | 30 |
| Isobutane | 22 |

**Table 4 Composition of Filling Prescription**

| Filling prescription for propellant | Role | Prescription ratio (wt%) | Dosage per can (g) |
|---|---|---|---|
| Content | content | 95 | 57 |
| Mixed alkanes | propellant | 5 | 3 |

2) The preparation process was as follows:

### (1) Liquid preparation

Preparing aqueous phase: purified water was weighed according to the prescribed amount, anhydrous citric acid was then added and the mixture was stirred until completely dissolved, thereby obtaining the aqueous phase;

Preparing alcoholic phase: glycerol, Tween 60, lactic acid, dibutyl hydroxytoluene, cetyl alcohol, and stearyl alcohol were added into anhydrous ethanol according to the prescribed amount, and the mixture was stirred at 35°C-60°C, then minoxidil was added and dispersed by continuous stirring, thereby obtaining the alcoholic phase;
mixing: the aqueous phase was added into the alcoholic phase, and stirred until minoxidil was completely dissolved, resulting in homogeneous mixing of both phases.

### (2) Filtering

The mixed solution was filtered through a filter cartridge.

### (3) Filling

The filtrate was filled into the aluminum spray cans (57g/can).

### (4) Sealing

Valves were installed and sealed.

### (5) Gas-charged

The mixed alkanes were filled into the cans (3g/can).

(6) The mousse nozzle cap and outer cap were mounted, followed by packaging, thereby obtaining the final product.

### Preparation of blank foam formulation:

The process steps were consistent with those for the 5% minoxidil foam formulation (specification: 5%), except that the addition of minoxidil was omitted from the alcoholic phase. Minoxidil was removed from the prescription, the dosage of anhydrous ethanol was 34.34 g/can and the prescription amount was adjusted to 60.24% (excluding propellant), the dosage of purified water was 19.54 g/can and the prescription amount was adjusted to 34.28% (excluding propellant), while the contents of other components were unchanged.

### Preparation of minoxidil-finasteride foam formulation (specification 5% -0.075%):

1) The prescription of composition is shown in Table 5

**Table 5 Prescription of Composition**

| component | Mass per can (g) | Proportion (wt%) (excluding propellant) |
|---|---|---|
| Minoxidil | 2.850 | 5.00 |
| Finasteride | 0.043 | 0.075 |
| Anhydrous ethanol | 32.182 | 56.46 |
| Glycerol | 1.203 | 2.11 |
| Tween-60 | 0.239 | 0.42 |
| lactic acid | 0.599 | 1.05 |
| BHT | 0.057 | 0.10 |
| Cetyl alcohol | 0.661 | 1.16 |
| Stearyl alcohol | 0.302 | 0.53 |
| Anhydrous citric | 0.063 | 0.11 |
| Purified water | 18.801 | 32.985 |
| Propellant | 3.000 | N/A |
| Total | 60.000 | 100.00 |

The composition of the propellant prescription and the filling prescription are shown in Tables 6 and 7.

**Table 6 Composition of Propellant Prescription**

| Composition of propellant (mixed alkanes) | Proportion (%) |
|---|---|
| Propane | 48 |
| Butane | 30 |
| Isobutane | 22 |

**Table 7 Composition of Filling Prescription**

| Filling prescription for propellant | Role | Prescription ratio (wt%) | Dosage per can (g) |
|---|---|---|---|
| Content | content | 95 | 57 |
| Mixed alkanes | propellant | 5 | 3 |

2) The preparation process was as follows:

### (1) Liquid preparation

Preparing aqueous phase: purified water was weighed according to the prescribed amount, anhydrous citric acid was then added and the mixture was stirred until it was completely dissolved, thereby obtaining the aqueous phase;

Preparing alcoholic phase:
Anhydrous ethanol was weighed according to the prescribed amount; finasteride was dissolved in a small amount of anhydrous ethanol;
Glycerol, Tween 60, lactic acid, finasteride ethanol solution, dibutyl hydroxytoluene, cetyl alcohol, and stearyl alcohol were added into the remaining anhydrous ethanol according to the prescribed amount, and the mixture was stirred at 35°C- 60°C, then minoxidil was added and dispersed by continuous stirring, thereby obtaining the alcoholic phase;
mixing: the aqueous phase was added into the alcoholic phase, followed by stirring until minoxidil was completely dissolved, resulting in homogeneous mixing of both phases.

### (2) Filtering

The mixed solution was filtered through a filter cartridge.

### (3) Filling

The filtrate was filled into the aluminum spray cans (57g/can).

### (4) Sealing

Valves were installed and sealed.

### (5) Gas-charged

The mixed alkanes were filled into the cans (3g/can).

(6)The mousse nozzle cap and outer cap were mounted, followed by packaging, thereby obtaining the final product.

### Preparation of minoxidil-finasteride foam formulation (specification 5% -0.008%):

The process steps were consistent with those of minoxidil-finasteride foam formulation (specification: 5% -0.075%), wherein the amount of finasteride was 0.005 g/can, and the weight content was 0.008% without propellant. The amount of purified water was adjusted accordingly, and the contents of other components were kept unchanged.

### Preparation of minoxidil-finasteride foam formulation (specification 5% -0.025%):

The process steps were consistent with those of minoxidil-finasteride foam formulation (specification: 5% -0.075%), wherein the amount of finasteride was 0.014 g/can, and the weight content was 0.025% without propellant. The amount of purified water was adjusted accordingly, and the contents of other components were kept unchanged.

### 1.2 Experimental Process and Results

C57BL/6J male mice were housed in a single cage in an SPF grade clean room with constant temperature and humidity laminar flow. After one week of adaptation, hair removal cream was used to remove approximately 3 × 4 cm² from their back. The skin turned pink and was undamaged, confirming that the mouse hair follicles were in the resting phase. The mice were divided randomly into 7 groups, as shown in Table 8 below.

**Table 8 Experimental Grouping**

| Group | Administration |
|---|---|
| Blank control group | Corn oil + Blank foam formulation |
| Model group | Testosterone propionate solution +Blank foam formulation |
| Minoxidil group | Testosterone propionate solution + 5% Minoxidil foam formulation |
| Finasteride group | Testosterone propionate solution + Oral administration of 0.01% finasteride (0.1 mg/kg) |
| Experimental group I | Testosterone propionate solution + Minoxidil-finasteride foam formulation (specification 5% -0.008%) |
| Experimental group II | Testosterone propionate solution + Minoxidil-finasteride foam formulation (specification 5% -0.025%) |
| Experimental group III | Testosterone propionate solution + Minoxidil-finasteride foam formulation (specification 5% -0.075%) |

Except for the blank control group, in which mice was injected with corn oil at multiple points every day, mice in each other group were subcutaneously injected with 30 mg/kg/day of testosterone propionate solution in the depilation area of the back skin (to establish an androgenic alopecia model). 0.5 hour after testosterone propionate injection, 200 mg of foam formulation was applied to the hair removal area on the back of mice in each foam formulation group, once a day for 30 consecutive days. The blank control group and the model group were given blank foam formulation once a day for 30 consecutive days.

The finasteride group was administered orally, with an initial dosage of 0.1 ml per mouse. The dosage was adjusted weekly based on changes in body weight, and the initial weight/0.1=weekly body weight/finasteride dosage, once a day for 30 consecutive days.

**The combination of active components with specific content in the present invention has fast onset and good effects**

### Hair Growth Score

The skin and hair growth at depilated sites of mice in each group were observed daily. The scores were assessed once weekly for the first two weeks, and twice weekly from the third week onward. Photographs were taken and the hair regrowth score results were recorded for each group during scoring session.

The scoring criteria are shown in Table 9.

**Table 9 Hair Growth Scoring Criteria**

| Hair growth status | Score |
|---|---|
| Not growing, the skin in the depilated area is pink | 0 point |
| The skin in the depilation area appears gray (with less than 20% growth) | 1 point |
| The skin in the depilation area appears black (greater than 20% but less than 40% growth) | 2 point |
| The skin in the depilation area appears black with slight hair growth (greater than 40% but less than 60% growth) | 3 point |
| The skin in the depilation area appears black with some hair growth (60% - 80% growth) | 4 point |
| Hair growth in the depilation area is essentially complete (80% - 100% growth) | 5 point |

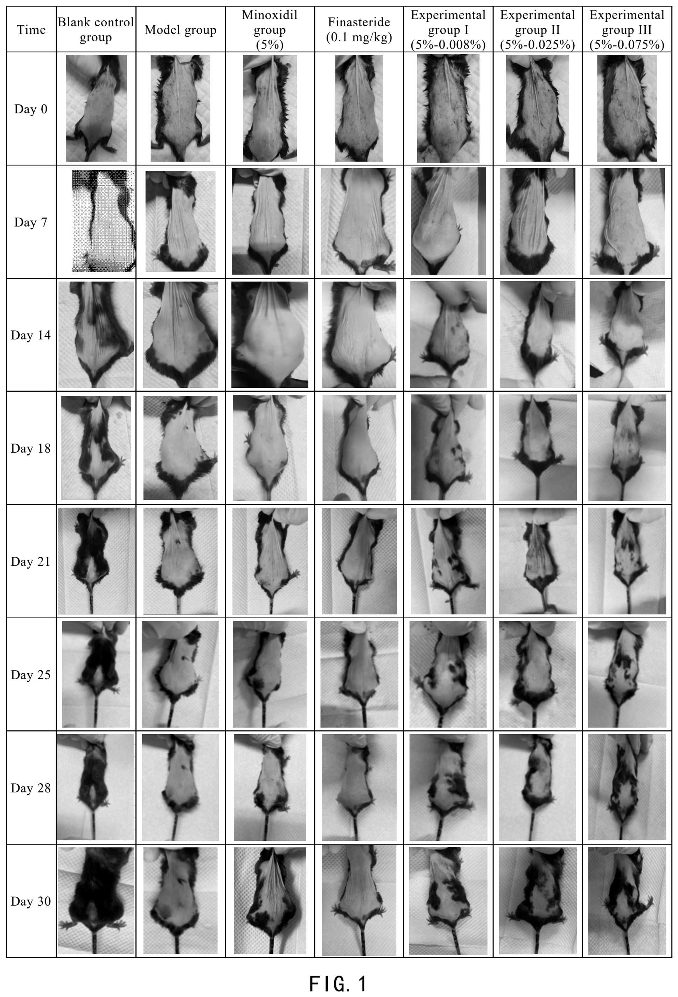

The scoring results are shown in Table 10 and FIG. 1.

**Table 10 Results of hair growth scores for each experimental group**

| Group | Score (mean ± SD) | | | | | | | |
|---|---|---|---|---|---|---|---|---|
| | Day 0 | Day 7 | Day 14 | Day 18 | Day 21 | Day 25 | Day 28 | Day 30 |
| Blank control group | 0±0 | 0.8±0.4 | 1.8±0.7 **## | 3.3±0.8 **## | 3.8±0.5 **## | 4.8±0.5, **## | 4.8±0.5 **## | 4.9±0.3 **## |
| Model group | 0±0 | 0.7±0.5 | 0.7±0.5 | 0.8±0.5 | 0.8±0.5 | 0.8±0.4 | 1.0±0.4 | 1.1±0.7 |
| Minoxidil group (5%) | 0±0 | 0.8±0.5 | 0.9±0.3 | 1.3±0.5 # | 1.5±0.8 ## | 1.9±1.0 *## | 2.3±1.3 *## | 2.3±1.3 *# |
| Finasteride group (0.1 mg/kg) | 0±0 | 0.7±0.5 | 0.8±0.5 | 0.9±0.7 | 1.0±0.6 | 1.2±0.4 # | 1.3±0.6 | 1.3±0.8 |
| Experimental group I (5%-0.008%) | 0±0 | 0.4±0.5 | 0.8±0.5 | 1.1±0.7 | 1.3±0.9 | 1.5+0.8 # | 1.8±0.8 ## | 1.8±0.8 # |
| Experimental group II (5%-0.025%) | 0±0 | 0.7±0.5 | 1.2±0.4 *#+ | 1.8±1.1 **##+ | 1.8±1.1 *##+ | 1.9±1.1 *##+ | 2.3±1.2 **##+ | 2.3±1.2 *##+ |
| Experimental group III (5%-0.075%) | 0±0 | 0.6±0.5 | 1.0±0.0 # | 1.8±0.8 **##++ | 2.5±1.4 **##&++ | 2.7±1.4 **##++ | 2.9±1.4 **##++ | 3.1±1.6 **##++ |

| | | | | | | | | |
|---|---|---|---|---|---|---|---|---|
| Note: Through one-way ANOVA, compared with the model group, * p<0.05, ** p<0.01; through T-test, compared with the model group, # p<0.05, ## p<0.01; compared with the minoxidil group & p<0.05; and compared with the oral finasteride group + p<0.05, ++ p<0.01. | | | | | | | | |

The results showed that there was no significant difference in the scoring results between the finasteride group and the model group. The scoring results of experimental group III were not only significantly higher than those of the minoxidil group, but also significantly higher than those of experimental group I and experimental group II groups.

Surprisingly, experimental group III showed extremely excellent effects.

On the one hand, on Day 18 after administration, the score results rapidly increased, and on Day 21, a significant difference existed as compared to the experimental group I and experimental group II. Taking the data on Day 21 as an example, the difference between experimental group III and the model group was 1.7, whereas the differences for the minoxidil group, finasteride group, experimental group I, and experimental group II as compared to the model group were 0.7, 0.2, 0.5, and 1, respectively. Therefore, the difference (or effect) between experimental group III and the model group was 2.4 times, 8.5 times, 3.4 times, and 1.7 times higher than that in the minoxidil group, finasteride group, experimental group I, and experimental group II, respectively.

On the other hand, this significant difference persisted until the end of this experiment (30 days after administration).

It can be seen that the onset time of minoxidil-finasteride foam formulation in experimental group III was significantly better than that of experimental group I, experimental group II and minoxidil single foam formulation group.

### Hair weight evaluation

After the experiment was completed, the experimental mice were euthanized, the hair on the skin surface of the depilated area of the mice was removed and weighed. The experimental results are shown in Table 11.

**Table 11 Statistical results of hair weight in each experimental group**

| Group | Hair weight (mg) (mean ± SD) | Difference as compared with model group (mg) (Δ) |
|---|---|---|
| Blank control group | 81.2±14.1 **## | 76.9 |
| Model group | 4.3±7.1 | 0 |
| Minoxidil group (5%) | 18.8±18.9# | 14.5 |
| Finasteride group (0.1 mg/kg) | 3.5±6.7 | -0.8 |
| Experimental group I (5% -0.008%) | 15.3±17.6+ | 11 |
| Experimental group II (5% -0.025%) | 24.1±26.9#+ | 19.8 |
| Experimental group III (5% -0.075%) | 36.7±33.8**##++ | 32.4 |

| | | |
|---|---|---|
| Note: Through one-way ANOVA, compared with the model group, ** p<0.01; through T-test, compared with the model group, # p<0.05, ## p<0.01; compared with the oral finasteride group, + p<0.05, ++ p<0.01. | | |

The results showed that there was no significant difference in hair weight between the finasteride group and the model group. Surprisingly, the hair weight of experimental group III was not only significantly greater than that of the minoxidil group, but also significantly greater than that of experimental groups I and II.

According to the difference with the model group, it can be seen that compared with the model group, the minoxidil group significantly increased hair weight, while the hair weight of the finasteride group did not increase but slightly decreased. The effect of experimental group I was worse than that of minoxidil group.

Surprisingly, the experimental group II showed a significant improvement in efficacy compared to the minoxidil group. Surprisingly, the hair growth effect of experimental group III (Δ=32.4) was about 2.3 times of that in the minoxidil group. And its effect on hair weight growth was significantly better than that in the group treated with finasteride alone, the group treated with minoxidil alone, and the combined effect of the group treated with minoxidil alone and the group treated with finasteride alone (i.e., Δ=14.3+(-0.8)).

It can be seen that a specific ratio of minoxidil and finasteride (e.g., 5% -0.075%) has a synergistic effect.

### Evaluation of hair follicle quantity

The skin was sampled parallel to the spine in the depilated area, trimmed into 1 cm-wide strips, fixed in 10% neutral buffered formalin for 24-36 hours, dehydrated through gradient ethanol, embedded in paraffin, sectioned after trimming, and stained with H&E. The sections were observed under an optical microscope, and the number of hair follicles was counted. The experimental results are shown in Table 12.

**Table 12 Statistical results of the number of hair follicles in each experimental group**

| Group | Number of hair follicles (hair follicles/100 µm) (mean ± SD) | Difference as compared with the model group (hair follicles/100 µm)(Δ) |
|---|---|---|
| Blank control group | 35±12** | 17 |
| Model Group | 18±8 | 0 |
| Minoxidil group (5%) | 25±9**## | 7 |
| Finasteride group (0.1 mg/kg) | 15±3 | -3 |
| Experimental group I (5% -0.008%) | 20±8++ | 2 |
| Experimental group II (5% -0.025%) | 22±6##++ | 4 |
| Experimental group III (5% -0.075%) | 28±12**##++ | 10 |

| | | |
|---|---|---|
| Note: Through one-way ANOVA, compared with the model group, ** p<0.01; through T-test, compared with the model group, ## p<0.01; compared with the oral finasteride group, + p<0.05, ++ p<0.01. | | |

The results showed that there was no significant difference in the number of hair follicles between the finasteride group and the model group, and even a slight decrease; and there was no significant difference between experimental group I, experimental group II, and minoxidil group, and the number of hair follicles in experimental group I and experimental group II was less than that in the minoxidil group.

Surprisingly, the number of hair follicles in experimental group III (Δ=10) was higher than that in the minoxidil group, and significantly higher than that in experimental groups I and II. Its effect on increasing the number of hair follicles was significantly better than that in the group treated with finasteride alone, the group treated with minoxidil alone, and the combined effect of the group treated with minoxidil alone and the group treated with finasteride alone (Δ=7+(-3)).

It can be seen that a specific ratio of minoxidil and finasteride (e.g., 5% -0.075%) has a synergistic effect.

### Example 2 Pharmacodynamic Study 2

### 2.1 Experimental reagents and materials

The experimental materials for this example are shown in Table 13.

**Table 13 Experimental materials**

| Experimental materials | Source |
|---|---|
| C57BL/6J male mice (6-8 week-aged) | SPF (Beijing) Biotechnology Co., Ltd. |
| Testosterone propionate | Shanghai Macklin Biochemical Co., Ltd. |
| Corn oil for injection | Sigma |
| Blank foam formulation | Self-prepared in this example |
| Minoxidil foam formulation (Specification 5%) | Self-prepared in this example |
| Minoxidil-finasteride foam formulation (Specification 5% -0.05%) | Self-prepared in this example |
| Minoxidil-finasteride foam formulation (Specification 5% -0.06%) | Self-prepared in this example |
| Minoxidil-finasteride foam formulation (Specification 5% -0.07%) | Self-prepared in this example |

### Preparation of testosterone propionate solution:

80.65 mg of testosterone propionate was accurately weighed and dissolved in 26.346 mL of injectable corn oil. Vortex-sonication was applied to obtain a homogeneous solution with a concentration of 3 mg/ml.

### Preparation of 5% minoxidil foam formulation:

The preparation method was the same as that for 5% minoxidil foam formulation in Example 1.

### Preparation of blank foam formulation:

The preparation method was the same as that for blank foam formulation in Example 1.

### Preparation of minoxidil-finasteride foam formulation (specification 5% -0.05%):

The process was consistent with that for the minoxidil-finasteride foam formulation (specification 5% - 0.075%) described in Example 1, wherein the finasteride dosage was set at 0.029 g per can, a mass fraction of 0.05% was achieved under propellant-free conditions, the amount of purified water was adjusted accordingly, and the contents of other were kept unchanged.

### Preparation of minoxidil-finasteride foam formulation (specification 5% -0.06%):

The process was consistent with that for the minoxidil-finasteride foam formulation (specification 5% - 0.075%) described in example 1, wherein the finasteride dosage was set at 0.034 g per can, a mass fraction of 0.06% was achieved under propellant-free conditions, the amount of purified water was adjusted accordingly, and the contents of other components were kept unchanged.

### Preparation of minoxidil-finasteride foam formulation (specification 5% -0.07%):

The process was consistent with that for the minoxidil-finasteride foam formulation (specification 5% - 0.075%) described in Example 1, wherein the finasteride dosage was set at 0.04 g per can, a mass fraction of 0.07% was achieved under propellant-free conditions, the amount of purified water was adjusted accordingly, and the contents of other components were kept unchanged.

### 2.2 Experimental Process and Results

C57BL/6J male mice were housed in a single cage in an SPF grade clean room with constant temperature and humidity laminar flow. After one week of adaptation, hair removal cream was used to remove approximately 3 × 4 cm² from their back. The skin turned pink and was undamaged, confirming that the mouse hair follicles were in the resting phase. The mice were divided into 6 groups randomly, as shown in table 14.

**Table 14 Experimental Grouping**

| Group | Administration |
|---|---|
| Blank control group | Corn oil + Blank foam formulation |
| Model group | Testosterone propionate solution + Blank foam formulation |
| Minoxidil group | Testosterone propionate solution + 5% Minoxidil foam formulation |
| Experimental group I | Testosterone propionate solution + Minoxidil-finasteride foam formulation (specification 5% -0.05%) |
| Experimental group II | Testosterone propionate solution + Minoxidil-finasteride foam formulation (specification 5% -0.06%) |
| Experimental group III | Testosterone propionate solution + Minoxidil-finasteride foam formulation (specification 5% -0.07%) |

Except for the blank control group, in which mice was injected with corn oil at multiple points every day, mice in each other group were subcutaneously injected with 30 mg/kg per day of testosterone propionate solution in the depilation area of the back skin (to establish an androgenic alopecia model). 0.5 hour after testosterone propionate injection, 200 mg of foam formulation was applied to the hair removal area on the back of mice in each foam formulation group, once a day for 30 consecutive days. The blank control group and the model group were given blank foam formulation once a day for 30 consecutive days.

**The combination of active components with specific content in the present invention has fast onset and good effects**

### Hair Growth Score

The skin and hair growth at depilated sites of mice in each group were observed daily. The scores were assessed once weekly for the first two weeks, and twice weekly from the third week onward. Photographs were taken and the hair regrowth score results were recorded for each group during scoring session. The scoring criteria are shown in Table 8.

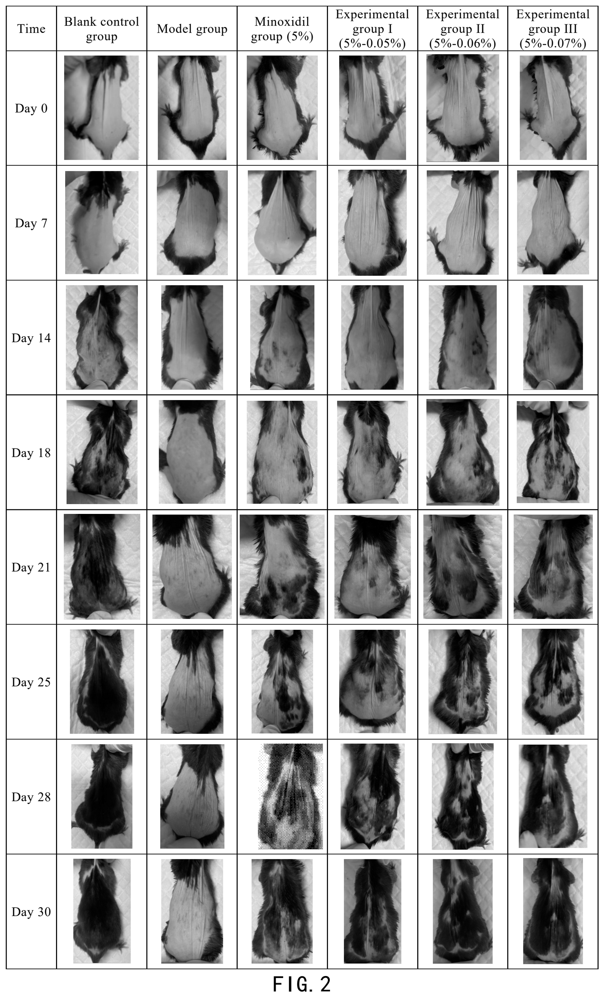

The scoring results are shown in Table 15 and FIG.2.

**Table 15 Results of hair growth scores for each experimental group**

| Group | Score (mean ± SD) | | | | | | | |
|---|---|---|---|---|---|---|---|---|
| | Day 0 | Day 7 | Day 14 | Day 18 | Day 21 | Day 25 | Day 28 | Day 30 |
| Blank control group | 0±0 | 0.9±0.4 # | 2.0±0 **## | 2.6±0.5 **## | 3.6±0.5 **## | 4.6±0.5 **## | 4.9±0.4 **## | 4.9±0.4 **## |
| Model group | 0±0 | 0.4±0.5 | 0.6±0.5 | 0.8±0.5 | 0.9±0.4 | 1.0±0 | 1.0±0 | 1.0±0 |
| Minoxidil group (5%) | 0±0 | 0.9±0.4 # | 1.1±0.6 | 1.4±0.7 | 1.9±0.8 *## | 2.1±0.8 ## | 2.3±1.0 *## | 2.4±1.2 *## |
| Experimental group I (5%-0.05%) | 0±0 | 0.4±0.5 | 1.0±0.8 | 1.4±0.5 # | 1.8±0. 9# | 2.3±1.2 ## | 2.5±1.2 *## | 2.6±1.4 **## |
| Experimental group II (5%-0.06%) | 0±0 | 0.5±0.5 | 1.1±0.6 | 1.5±1.1 | 1.9±0.8 *## | 2.3±1.0 *## | 2.5±1.1 *## | 2.6±1.1 *## |
| Experimental group III (5%-0.07%) | 0±0 | 0.8±0.5 | 1.3±0.7 | 2.0±0.5 **## | 2.3±0.7 **## | 2.8±1.2 **## | 3.0±1.3 **## | 3.3±1.2 **## |

| | | | | | | | | |
|---|---|---|---|---|---|---|---|---|
| Note: Through one-way ANOVA, compared with the model group, * p<0.05, ** p<0.01; through T-test, compared with the model group, # p<0.05, ## p<0.01. | | | | | | | | |

The results showed that the scores of all groups were significantly higher than those of the model group. And the scoring results of experimental group III were not only significantly higher than those in the minoxidil group, but also significantly higher than those in experimental group I and experimental group II.

Surprisingly, experimental group III showed extremely excellent effects.

On the one hand, on Day 14 after administration, the score rapidly increased, and on Day 18, a significant difference was existed as compared to the experimental group I and experimental group II. The data on Day 18 were taken as an example, the difference between the experimental group III and the model group was 1.2, the differences between the minoxidil group or the experimental group I and the model group were 0.6, and the difference between the experimental group II and the model group was 0.7. Therefore, the difference (or effect) between the experimental group III and the model group was twice higher than that in the minoxidil group alone or the experimental group I, and 1.7 times higher than that in the experimental group II.

On the other hand, this significant trend continued until the end of this experiment (30 days after administration).

It can be seen that the onset time of minoxidil-finasteride foam formulation in experimental group III was significantly better than that in experimental group I, experimental group II and minoxidil single foam formulation group.

### Hair weight evaluation

After the experiment was completed, the experimental mice were euthanized, the hair on the skin surface of the depilated area of the mice was removed, and weighed. The experimental results are shown in Table 16.

**Table 16 Statistical results of hair weight in each experimental group**

| Group | Hair weight (mg) (mean ± SD) | Difference as compared with model group (mg) (Δ) |
|---|---|---|
| Blank control group | 86.4±15**## | 81.5 |
| Model group | 4.9±4.6 | 0 |
| Minoxidil group (5%) | 47.9±25.9**## | 43 |
| Experimental group I (5% -0.05%) | 51.3±31.4**## | 45.4 |
| Experimental group II (5% -0.06%) | 48.7±27.2**## | 43.8 |
| Experimental group III (5% -0.07%) | 67.9±33.2**## | 63 |

| | | |
|---|---|---|
| Note: Through one-way ANOVA, compared with the model group, ** p<0.01; through T-test, compared with the model group, ## p<0.01. | | |

The results showed that the hair weight in the minoxidil group, experimental groups I, II, and III was significantly greater than that in the model group; wherein, the hair weight in experimental group III was not only significantly greater than that in the minoxidil group, but also significantly greater than that in experimental groups I and II.

According to the difference with the model group, it can be seen that compared with the model group, the minoxidil group can significantly increase hair weight. The experimental effects in experimental group I and experimental group II were comparable to those in the minoxidil group.

Surprisingly, the experimental group III showed significant improvement compared to the minoxidil group, experimental group I, and experimental group II, and the hair growth effect in the experimental group III was about 1.5 times higher than that in the minoxidil group.

It can be seen that a specific ratio of minoxidil and finasteride (such as 5% -0.07%) can achieve extremely excellent effect improvement and has a synergistic effect.

### Evaluation of hair follicle quantity

The skin was sampled parallel to the spine in the depilated area, trimmed into 1 cm-wide strips, fixed in 10% neutral buffered formalin for 24-36 hours, dehydrated through gradient ethanol, embedded in paraffin, sectioned after trimming, and stained with H&E. The sections were observed under an optical microscope, and the number of hair follicles was counted. The experimental results are shown in table 17.

**Table 17 Statistical results of the number of hair follicles in each experimental group**

| Group | Number of hair follicles (hair follicles/100 µm) (mean ± SD) | Difference as compared with model group (mg) (Δ) |
|---|---|---|
| Blank control group | 37±7**## | 19 |
| Model group | 18±6 | 0 |
| Minoxidil group (5%) | 25±5# | 7 |
| Experimental group I (5% -0.05%) | 21±4 | 3 |
| Experimental group II (5% -0.06%) | 24±10 | 6 |
| Experimental group III (5% -0.07%) | 27±7# | 9 |

| | | |
|---|---|---|
| Note: Through one-way ANOVA, compared with the model group, ** p<0.01; through T-test, ## p<0.01, # p<0.05. | | |

The results showed that the number of hair follicles in the minoxidil group, experimental groups I, II, and III was higher than that in the model group; wherein, the number of hair follicles in experimental group III was not only higher than that in the minoxidil group, but also higher than that in experimental groups I and II.

Based on the differences as compared to the model group, it was observed that the minoxidil group significantly increased hair weight compared to the model group, while experimental group I also demonstrated a hair weight increase with a less extent than that in the minoxidil group; and experimental group II showed comparable effectiveness as compared with the minoxidil group.

Surprisingly, the effect of experimental group III was further improved compared to the minoxidil group, and the hair growth effect was about 1.3 times higher than that in the minoxidil group.

It can be seen that a specific ratio of minoxidil and finasteride (such as 5% -0.07%) can achieve extremely excellent effect improvement.

### Example 3 Pharmacodynamic Experiment 3

### 3.1 Experimental reagents and materials

The experimental materials for this example are shown in Table 18.

**Table 18 Experimental Materials**

| Experimental materials | Source |
|---|---|
| C57BL/6J male mice (6-8 week-aged) | SPF (Beijing) Biotechnology Co., Ltd. |
| Testosterone propionate | Shanghai Macklin Biochemical Co., Ltd. |
| Corn oil for injection | Sigma |
| Blank foam formulation | Self-prepared in this example |
| Minoxidil-finasteride foam formulation (Specification 5% -0.075%) | Self-prepared in this example |
| Minoxidil-finasteride foam formulation (Specification 5% -0.1%) | Self-prepared in this example |
| Minoxidil-finasteride foam formulation (Specification 5% -0.15%) | Self-prepared in this example |

### Preparation of testosterone propionate solution:

92.29 mg of testosterone propionate was weighed accurately and dissolved in 30.148 mL of corn oil for injection. Vortex-sonication was applied to obtain a homogeneous solution with a concentration of 3 mg/ml.

### Preparation of minoxidil-finasteride foam formulation (specification 5% -0.075%):

The preparation method was the same as the preparation for minoxidil-finasteride foam formulation (specification 5% -0.075%) in Example 1.

### Preparation of blank foam formulation:

The preparation method was the same as that for blank foam formulation in Example 1.

### Preparation of minoxidil-finasteride foam formulation (specification 5% -0.1%):

The process was consistent with that for the minoxidil-finasteride foam formulation (specification 5% - 0.075%) described in Example 1, wherein the finasteride dosage was set at 0.057 g per can, a mass fraction of 0.1% was achieved under propellant-free conditions, the amount of purified water was adjusted accordingly, and the contents of other components were kept unchanged.

### Preparation of minoxidil-finasteride foam formulation (specification 5% -0.15%):

The process was consistent with that for the minoxidil-finasteride foam formulation (specification 5% - 0.075%) described in Example 1, wherein the finasteride dosage was set at 0.086 g per can, a mass fraction of 0.15% was achieved under propellant-free conditions, the amount of purified water was adjusted accordingly, and the contents of other components were kept unchanged.

### 3.2 Experimental Process and Results

C57BL/6J male mice were housed in a single cage in an SPF grade clean room with constant temperature and humidity laminar flow. After one week of adaptation, hair removal cream was used to remove approximately 3 × 4 cm² from their back. The skin turned pink and was undamaged, confirming that the mouse hair follicles were in the resting phase. The mice were divided into 5 groups randomly, as shown in Table 19.

**Table 19 Experimental Grouping**

| Group | Administration |
|---|---|
| Blank control group | Corn oil + Blank foam formulation |
| Model group | Testosterone propionate solution + Blank foam formulation |
| Experimental group I | Testosterone propionate solution + Minoxidil-finasteride foam formulation (specification 5% -0.075%) |
| Experimental group II | Testosterone propionate solution + Minoxidil-finasteride foam formulation (specification 5% -0.1%) |
| Experimental group III | Testosterone propionate solution + Minoxidil-finasteride foam formulation (specification 5% -0.15%) |

Except for the blank control group, in which mice was injected with corn oil at multiple points every day, mice in each other group were subcutaneously injected with 30 mg/kg/day of testosterone propionate solution in the depilation area of the back skin (to establish an androgenic alopecia model). 0.5 hour after testosterone propionate injection, 200 mg of foam formulation was applied to the depilated area of the back of mice in each group once a day for 30 consecutive days. The blank control group and the model group were given blank foam formulation once a day for 30 consecutive days.

**The combination of active components with specific content in the present invention has fast onset and good effects**

### Hair Growth Score

The skin and hair growth at depilated sites of mice in each group were observed daily. The scores were assessed once weekly for the first two weeks, and twice weekly from the third week onward. Photographs were taken and the hair regrowth score results were recorded for each group during scoring session. The scoring criteria are shown in Table 8.

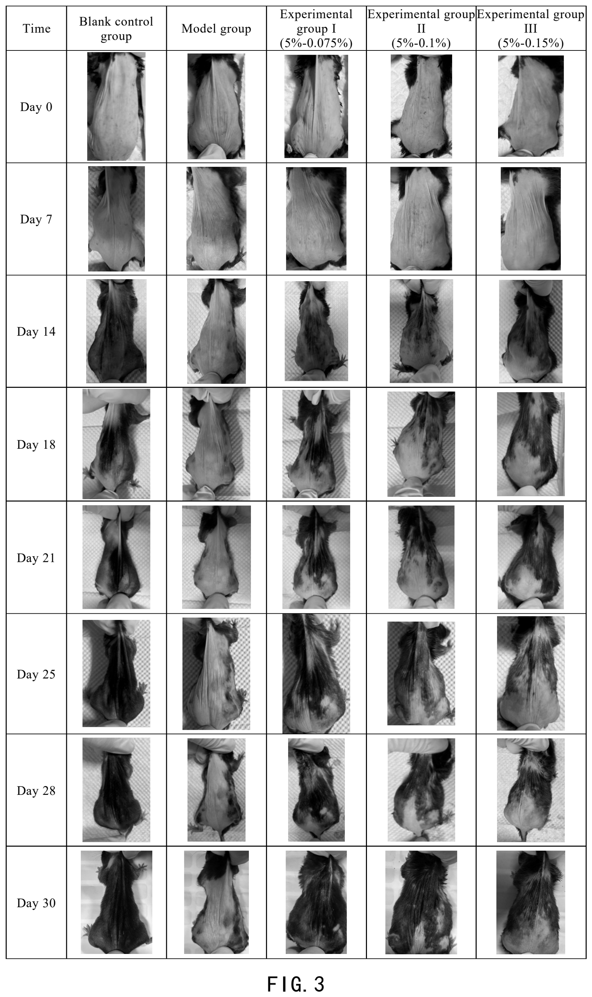

The scoring results are shown in Table 20 and FIG.3.

**Table 20 Results of hair growth scores for each experimental group**

| Group | Score (mean + SD) | | | | | | | |
|---|---|---|---|---|---|---|---|---|
| | Day 0 | Day 7 | Day 14 | Day 18 | Day 21 | Day 25 | Day 28 | Day 30 |
| Blank control group | 0±0 | 0.3+0.5 | 1.8±0.7 **## | 2.5±1.3 **## | 2.8±1.3 **## | 3.8±1.4 **## | 3.8±1.4 **## | 4.4±1.1 **## |
| Model group | 0±0 | 0±0 | 0.8±0.5 | 0.9±0.4 | 0.9±0.4 | 1±0.5 | 1.1±0.6 | 1.3±0.9 |
| Experimental group I (5%-0.075%) | 0±0 | 0.1±0.4 | 1.6±0.5 **## | 2.4±0.7 **## | 2.6±1.1 **## | 3±1.2 **## | 3.5±0.9 **## | 3.9±1 **## |
| Experimental group II (5%-0.1%) | 0±0 | 0.1±0.4 | 1.3±0.5 # | 1.9±0.8 ## | 2.1±0.6 *## | 2.6±0.9 *## | 2.9±0.8 **## | 3.6±1.1 **## |
| Experimental group III (5%-0.15%) | 0±0 | 0.3±0.5 | 1.3±0.5 # | 2±1.1 # | 2.3±1 *## | 3±1.2 **## | 3.4±1.3 **## | 3.8±1.5 **## |

| | | | | | | | | |
|---|---|---|---|---|---|---|---|---|
| Note: Through one-way ANOVA, compared with the model group, * p<0.05, ** p<0.01; through T-test, compared with the model group, # p<0.05, ## p<0.01. | | | | | | | | |

The results showed that the scores in all groups were significantly higher than those in the model group. And the scoring results of experimental group I were higher than those in experimental group II and experiment group III.

Surprisingly, experimental group I showed extremely excellent effects.

On the one hand, with a decreased dosage of finasteride, its score rapidly increased on Day 14 after administration, and on Day 18, a significant difference existed as compared with the experimental groups II and III. The data on Day 14 were taken as an example, the difference between experimental group I and the model group was 0.8, while the difference between experimental group II or experimental group III and the model group was 0.5. Therefore, the difference (or effect) between experimental group I and the model group was 1.6 times higher than that in experimental group II and experimental group III.

On the other hand, this significant trend continued until Day 21 of administration. From Day 25 to Day 30, the increasing trend in experimental group I and experimental group III was similar and higher than that in experimental group II.

It can be seen that the onset time of minoxidil-finasteride foam formulation in experimental group I was significantly better than that in experimental group II and experimental group III.

### Hair weight evaluation

After the experiment was completed, the experimental mice were euthanized, the hair on the skin surface of the depilated area of the mice was removed, and weighed. The experimental results are shown in Table 21.

**Table 21 Statistical results of hair weight in each experimental group**

| Group | Hair weight (mg) (mean ± SD) | Difference as compared with model group (mg) (Δ) |
|---|---|---|
| Blank control group | 78±30**## | 60 |
| Model group | 18±15 | 0 |
| Experimental group I (5% -0.075%) | 66±25**## | 48 |
| Experimental group II (5% -0.1%) | 57±28**## | 39 |
| Experimental group III (5% -0.15%) | 57±9**## | 39 |

| | | |
|---|---|---|
| Note: Through one-way ANOVA, compared with the model group, * p<0.05, ** p<0.01; through T-test, compared with the model group, # p<0.05, ## p<0.01. | | |

The results showed that the hair weight in experimental groups I, II, and III was significantly greater than that in the model group; wherein, the hair weight of experimental group I was greater than that in experimental groups II and III.

According to the difference with the model group, it can be seen that compared with the model group, the hair weight in experimental groups I-III has significantly increased.

Surprisingly, with a decrease in the dosage of finasteride, experimental group I showed a significant improvement compared to experimental groups II and III, and had a growth effect 1.2 times higher than that in experimental groups II and III.

It can be seen that a specific ratio of minoxidil and finasteride (e.g., 5% -0.075%) has a synergistic effect.

### Evaluation of hair follicle quantity

The skin was sampled parallel to the spine in the depilated area, trimmed into 1 cm-wide strips, fixed in 10% neutral buffered formalin for 24-36 hours, dehydrated through gradient ethanol, embedded in paraffin, sectioned after trimming, and stained with H&E. The sections were observed under an optical microscope, and the number of hair follicles was counted. The experimental results are shown in Table 22.

**Table 22 Statistical results of the number of hair follicles in each experimental group**

| Group | Number of hair follicles (hair follicles/100 µ m) (mean ± SD) | Difference as compared with model group (hair follicles/100 µm) (Δ) |
|---|---|---|
| Blank control group | 19±11# | 11 |
| Model group | 8±3 | 0 |
| Experimental group I (5% -0.075%) | 23±17*# | 15 |
| Experimental group II (5% -0.1%) | 19±7## | 11 |
| Experimental group III (5% -0.15%) | 19±8## | 11 |

| | | |
|---|---|---|
| Note: Through one-way ANOVA, compared with the model group, * p<0.05, ** p<0.01; through T-test, compared with the model group, # p<0.05, ## p<0.01. | | |

The results showed that the number of hair follicles in experimental groups I, II, and III was significantly greater than that in the model group.

Surprisingly, with a decrease in the dosage of finasteride, the number of hair follicles in experimental group I was significantly higher than that in experimental groups II and III, and even significantly higher than that in the blank control group.

It can be seen that a specific ratio of minoxidil and finasteride (such as 5% -0.075%) has extremely excellent effects.

### Example 4 Pharmacodynamic Study 4

### 4.1 Experimental preparations and materials

The experimental materials for this example are shown in Table 23.

**Table 23 Experimental Materials**

| Experimental materials | Source |
|---|---|
| C57BL/6J male mice (6-8 week-aged) | SPF (Beijing) Biotechnology Co., Ltd. |
| Testosterone propionate | Shanghai Macklin Biochemical Co., Ltd. |
| Corn oil for injection | Sigma |
| Blank liniment | Self-prepared in this example |
| Minoxidil-Finasteride liniment (Specification 5% -0.05%) | Self-prepared in this example |
| Minoxidil-Finasteride liniment (Specification 5% -0.75%) | Self-prepared in this example |
| Minoxidil-Finasteride liniment (Specification 5% -0.1%) | Self-prepared in this example |

### Preparation of testosterone propionate solution:

66.42 mg of testosterone propionate was weighed accurately and dissolved in 21.697 mL of corn oil for injection. Vortex-sonication was applied to obtain a homogeneous solution with a concentration of 3 mg/ml.

### Preparation of Minoxidil-Finasteride Liniment (Specification 5% -0.075%):

1) The prescription composition is shown in Table 24.

**Table 24 Prescription Composition**

| Prescription composition | Dosage/bottle | Ratio |
|---|---|---|
| Minoxidil | 3.00 g | 5%(w/v) |
| Finasteride | 0.45 g | 0.075%(w/v) |
| Propylene glycol | 30 ml | 50%(v/v) |
| Anhydrous ethanol | 18 ml | 30%(v/v) |
| Purified water | appropriate amount | N/A |
| Total | 60 ml | N/A |

2) The preparation process was as follows:
(1) Minoxidil, finasteride, propylene glycol, and anhydrous ethanol were weighed according to the above formulation;
(2) finasteride was dissolved in a small amount of anhydrous ethanol;
(3) propylene glycol in the prescription-specified quantity, the remaining anhydrous ethanol, and a portion of purified water were uniformly mixed by stirring;
(4) minoxidil and finasteride ethanol solution were added separately to the mixed solution obtained from (3), and the mixture was stirred at room temperature until the medicinal solution became clear and transparent;
(5) purified water was added and diluted to the prescribed amount to obtain the medicinal solution;
(6) the obtained medicinal solution was filtered by a 0.45 µm microporous membrane;
(7) the medicine solution was filled and packaged according to the specification of 60 ml/bottle.

### Preparation of blank liniment:

The operation process was the same as the preparation of minoxidil-finasteride liniment (specification 5% -0.075%) except that minoxidil and finasteride were removed from the process. Minoxidil and finasteride were removed from the prescription, while the contents of other components were kept unchanged.

### Preparation of Minoxidil-Finasteride Liniment (Specification 5% -0.05%):

The preparation process was consistent with that for the minoxidil-finasteride liniment (specification 5% -0.075%), wherein the proportion of finasteride was 0.05% (w/v), the dosage was 0.03g/bottle, and the amount of purified water was adjusted accordingly, while the contents of other components were kept unchanged.

### Preparation of Minoxidil-Finasteride Liniment (Specification 5% -0.1%):

The preparation process was consistent with that for minoxidil-finasteride liniment (specification 5% -0.075%), wherein the proportion of finasteride was 0.1% (w/v), the dosage was 0.06g/bottle, and the amount of purified water was adjusted accordingly, while the contents of other components were kept unchanged.

### 4.2 Experimental Process and Results

C57BL/6J male mice were housed in a single cage in an SPF grade clean room with constant temperature and humidity laminar flow. After one week of adaptation, hair removal cream was used to remove approximately 3 × 4 cm² from their back. The skin turned pink and was undamaged, confirming that the mouse hair follicles were in the resting phase. The mice were divided into 5 groups randomly, as shown in Table 25.

**Table 25 Experimental Grouping**

| Group | Administration |
|---|---|
| Blank control group | Corn oil + Blank liniment |
| Model group | Testosterone propionate solution + Blank liniment |
| Experimental group I | Testosterone propionate solution + Minoxidil-Finasteride liniment (Specification 5% -0.05%) |
| Experimental group II | Testosterone propionate solution + Minoxidil-Finasteride liniment (Specification 5% -0.075%) |
| Experimental group III | Testosterone propionate solution + Minoxidil-Finasteride liniment (Specification 5% -0.1%) |

Except for the blank control group, in which mice was injected with corn oil at multiple points every day, each other group of mice was subcutaneously injected with 30 mg/kg/day of testosterone propionate solution in the depilation area of the back skin. After 0.5 hours of testosterone propionate injection, 0.2 ml of liniment was applied to the depilation area of the back of each group of mice once a day for 30 consecutive days. The blank control group and the model group were given blank liniment once a day for 30 consecutive days.

**The combination of active components with specific content in the present invention has fast onset and good effects**

### Hair Growth Score

The skin and hair growth at depilated sites of mice in each group were observed daily, and the effect of drugs on the hair growth cycle of mice was evaluated. The scores were assessed once weekly for the first two weeks, and twice weekly from the third week onward. Photographs were taken and the hair regrowth score results were recorded for each group during scoring session. The scoring criteria are shown in Table 8.

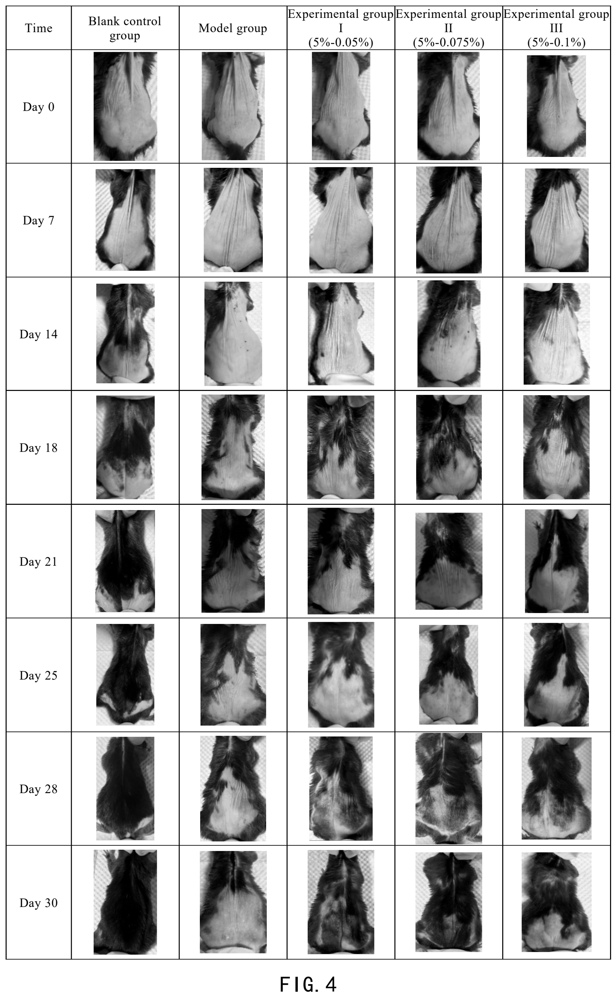

The scoring results are shown in Table 26 and FIG.4

**Table 26 Results of hair growth scores for each experimental group**

| Group | Score (mean ± SD) | | | | | | | |
|---|---|---|---|---|---|---|---|---|
| | Day 0 | Day 7 | Day 14 | Day 18 | Day 21 | Day 25 | Day 28 | Day 30 |
| Blank control group | 0±0 | 0±0 | 1.8±1 **## | 2.3±1.4 **# | 3.2±1.2 **## | 3.5+1 | 4.8±0.4 **## | 5±0 **## |
| Model group | 0±0 | 0±0 | 0.1±0.4 | 0.8±0.7 | 0.9±0.6 | 0.9±0.6 | 1.4±1.2 | 1.9±1.5 |
| Experimental group I (5%-0.05%) | 0±0 | 0±0 | 0.4±0.5 | 1.1±0.6 | 1.9±0.8 # | 2.1±0.8 *## | 3±1.3 *# | 3.3±1.2 * |
| Experimental group II (5%-0.075%) | 0±0 | 0±0 | 0.8±0.7 # | 1.9±0.8 *# | 2.1±0.8 *## | 2.3±0.9 *## | 2.9±0.8 *# | 3.4±0.7 *# |
| Experimental group III (5%-0.1%) | 0±0 | 0±0 | 0.5±0.5 | 1.3±0.5 | 1.8±0.7 # | 1.9±0.6 ## | 2.8±0.7 # | 3.1±1 |

| | | | | | | | | |
|---|---|---|---|---|---|---|---|---|
| Note: Through one-way ANOVA, compared with the model group, * p<0.05, ** p<0.01; through T-test, compared with the model group, # p<0.05, ## p<0.01. | | | | | | | | |

The results showed that the scores in all groups were significantly higher than those in the model group. The scoring results of experimental group II were significantly higher than those in experimental group I and experimental group III.

Surprisingly, experimental group II showed extremely excellent effects.

On the one hand, with a decreased dosage of finasteride, its score rapidly increased on Day 14 after administration, and on Day 18, a significant difference existed as compared with the experimental groups I and III. The data on Day 18 were taken as an example, the difference between experimental group II and the model group was 1.1, the difference between experimental group I and the model group was 0.3, and the difference between experimental group III and the model group was 0.5. Therefore, the difference (or effect) between experimental group II and the model group was 3.6 times higher than that in experimental group I and 2.2 times higher than that in experimental group III.

On the other hand, this trend continued until Day 21 of administration. Afterwards, the three groups showed similar upward trends.

From this, it can be seen that the onset time of minoxidil-finasteride liniment in experimental group II is significantly better than that in experimental groups I and III.

### Hair weight evaluation

After the experiment was completed, the experimental mice were euthanized, the hair on the skin surface of the depilated area of the mice was removed and weighed. The experimental results are shown in Table 27.

**Table 27 Statistical results of hair weight in each experimental group**

| Group | Hair weight (mg) (mean ± SD) | Difference as compared with model group (mg) (Δ) |
|---|---|---|
| Blank control group | 88±10**## | 70 |
| Model group | 18±14 | 0 |
| Experimental group I (5% -0.05%) | 38±26 | 20 |
| Experimental group II (5% -0.075%) | 44±16*## | 26 |
| Experimental group III (5% -0.1%) | 38±13# | 20 |

| | | |
|---|---|---|
| Note: Through one-way ANOVA, compared with the model group, * p<0.05, ** p<0.01; through T-test, compared with the model group, # p<0.05, ## p<0.01. | | |

The results showed that the hair weight in experimental groups I, II, and III was significantly higher than that in the model group, wherein the hair weight of experimental group II was greater than that in experimental groups I and III.

According to the difference with the model group, it can be concluded that experimental groups I-III can significantly increase hair weight compared to the model group. Surprisingly, although the dosage of finasteride in experimental group II was lower than that in experimental group III, its effect was 1.3 times higher than that in experimental group III.

It can be seen that a specific ratio of minoxidil and finasteride (e.g., 5% -0.075%) has a synergistic effect.

### Evaluation of hair follicle quantity

At the end of the experiment, animal skin was collected, fixed in formalin, and subjected to H&E staining. Four fields of view were selected for hair follicle counting. The experimental results are shown in Table 28.

**Table 28 Statistical results of the number of hair follicles in each experimental group**

| Group | Number of hair follicles (hair follicles/100 µ m) (mean ± SD) | Difference as compared with model group (hair follicles/100 µm) (Δ) |
|---|---|---|
| Blank control group | 41±19*## | 26 |
| Model group | 15±6 | 0 |
| Experimental group I (5% -0.05%) | 31±14# | 16 |
| Experimental group II (5% -0.075%) | 35±23# | 20 |
| Experimental group III (5% -0.1%) | 33±15## | 18 |

| | | |
|---|---|---|
| Note: Through one-way ANOVA, compared with the model group, * p<0.05, ** p<0.01; through T-test, compared with the model group, # p<0.05, ## p<0.01. | | |

The results showed that the number of hair follicles in experimental groups I, II, and III was significantly higher than that in the model group, wherein the number of hair follicles in experimental group II was greater than that in experimental groups I and III.

According to the difference with the model group, experimental groups I-III showed a significant increase in the number of hair follicles compared to the model group. The effect of experimental group II was better than that in experimental group I.

Surprisingly, although the dosage of finasteride in experimental group II was lower than that in experimental group III, its effect was 1.1 times higher than that in experimental group III.

Therefore, a specific ratio of minoxidil and finasteride (e.g., 5% -0.075%) has a synergistic effect.

### DISCUSSION

Minoxidil is a potassium channel agonist that can dilate local capillaries, increase local blood supply, and stimulate hair growth in the affected area. The marketed varieties include minoxidil solution (2%, 5%) and minoxidil foam formulation (5%). When using minoxidil solution, if local repeated itching and skin redness occur, one may try to use minoxidil without propylene glycol, that is, internationally recommended foaming preparation, to reduce or eliminate adverse allergic reactions. Topical application of minoxidil has a "shedding phase", and patients have poor compliance of use. The "shedding phase" refers to a phenomenon in which approximately 20% of alopecia patients experience transient shedding (a surge in temporary hair loss) during the initial 2-4 weeks of minoxidil therapy, with excessive shedding persisting for up to 1.5 months. Clinical studies have shown that after the onset of the "shedding phase", most patients experience emotional anxiety, and some patients may stop administration so that the symptoms of hair loss is further exacerbated.

Finasteride is a specific 5α - reductase inhibitor. 5α - reductase catalyzes the conversion of testosterone (T) to dihydrotestosterone (DHT), which can cause AGA when DHT accumulates to high levels in the skin. Finasteride treats androgenic alopecia by inhibiting the conversion of testosterone to dihydrotestosterone. The marketed varieties include finasteride tablet (1 mg) and finasteride spray (2.275 mg/mL). The adverse reactions of finasteride greatly affect the psychological compliance of patients when using it. Finasteride has good overall tolerance, and adverse reactions are usually mild, so treatment generally does not need to be discontinued. The main adverse events are decreased libido (1.9%), erectile dysfunction (1.3%), and reduced ejaculation (1%), with an incidence rate of less than 2%. Most adverse events gradually disappear after continuing treatment. Although finasteride has fewer adverse reactions, its potential impact on sexual dysfunction greatly limits its use for most male patients. The oral use of finasteride spray was changed to external use, and its effect was similar to that of oral finasteride, but the systemic exposure was significantly reduced, the effect on serum dihydrotestosterone concentration was small, and it had good tolerance. In the key phase III study, after local administration of finasteride at the expected dose (up to 200 microliters = up to 4 spray per day), the average maximum plasma finasteride concentration at all sampling times within 6 months of treatment was >100 times lower than the concentration after oral administration of 1 mg of finasteride per day.

Existing clinical trials have proved that topical minoxidil solution, topical minoxidil foam formulation, oral finasteride tablets, and topical finasteride spray are effective for androgenic alopecia. However, drug therapy still has many shortcomings, mainly manifested in slow onset and low efficacy. At present, the average onset time of minoxidil is 6-9 months, with an effective rate of only 60%. The overall effective rate of finasteride tablets in the treatment of mild and moderate AGA can reach 68% -77%, with the effective rates of mild improvement, moderate improvement, and significant improvement being 38.04%, 23.37%, and 13.59%, respectively. The overall treatment effect lasts for 6-9 months, and the efficacy is positively correlated with the duration of medication.

Minoxidil generates hair and finasteride stops shedding, forming a complementary pharmacological mechanism. According to the "Chinese Guideline for the Diagnosis and Treatment of Androgenetic Alopecia (2023)", the combination therapy of finasteride and minoxidil scored 0.80 points higher than treatment with minoxidil alone. The compound preparation of minoxidil-finasteride lipid solution for topical use (3% minoxidil + 0.1% finasteride, 5% minoxidil + 0.1% finasteride, 10% minoxidil + 0.1% finasteride) has been launched in India. Globally, no minoxidil-finasteride foam formulation has been launched yet.

Finasteride is not recommended for women to use. The specification of minoxidil for men is 5%. With a fixed specification of 5% minoxidil, the present invention reduces the amount of finasteride and minimizes side effects. At the same time, under specific ratios, the effect is fast and effective.

The experimental results of the present invention fully demonstrate that when the content of minoxidil is 5% and the content of finasteride is 0.075%, it has an extremely excellent effect on promoting hair growth, can effectively treat alopecia, and has a synergistic effect at this specific ratio.

All references mentioned in the present invention are cited as references in this application, as if each reference were cited separately. In addition, it should be understood that after reading the above teaching content of the present invention, those skilled in the art can make various modifications or modifications to the present invention, and these equivalent forms also fall within the scope of the claims attached to this application.

## Claims

1. A formulation or composition for treating alopecia, comprising the following active components on the basis of the mass percentage (w/w) or mass/volume percentage (w/v):
(a) 5% minoxidil; and
(b) 0.07-0.095% finasteride.

2. The formulation or composition of claim 1, comprising the following active components on the basis of the mass percentage (w/w) or mass/volume percentage (w/v):
(a) 5% minoxidil; and
(b) 0.07-0.09% finasteride;
preferably, comprising the following active components on the basis of the mass percentage (w/w) or mass/volume percentage (w/v):
(a) 5% minoxidil; and
(b) 0.07-0.085% finasteride;
preferably, comprising the following active components on the basis of the mass percentage (w/w) or mass/volume percentage (w/v):
(a) 5% minoxidil; and
(b) 0.07-0.08% finasteride;
more preferably, comprising the following active components on the basis of the mass percentage (w/w) or mass/volume percentage (w/v):
(a) 5% minoxidil; and
(b) 0.07-0.075% finasteride.

3. The formulation or composition of claim 1, comprising the following active components on the basis of the mass percentage (w/w) or mass/volume percentage (w/v):
(a) 5% minoxidil; and
(b) 0.075% finasteride.

4. The formulation or composition of any of claims 1-3, wherein the formulation or composition is a foam formulation or a liniment formulation.

5. A foam formulation for treating alopecia, comprising the following active components on the basis of the mass percentage (w/w):
(a) 5% minoxidil; and
(b) 0.07-0.095% finasteride;
preferably, comprising the following active components on the basis of the mass percentage:
(a) 5% minoxidil; and
(b) 0.07-0.09% finasteride;
preferably, comprising the following active components on the basis of the mass percentage:
(a) 5% minoxidil; and
(b) 0.07-0.085% finasteride;
preferably, comprising the following active components on the basis of the mass percentage:
(a) 5% minoxidil; and
(b) 0.07-0.08% finasteride;
preferably, comprising the following active components on the basis of the mass percentage:
(a) 5% minoxidil; and
(b) 0.07-0.075% finasteride;
more preferably, comprising the following active components on the basis of the mass percentage:
(a) 5% minoxidil; and
(b) 0.075% finasteride;
wherein the total mass in the mass percentage does not contain the mass of a propellant.

6. The foam formulation of claim 5, wherein the foam formulation further comprises a component selected from the group consisting of a humectant, a solubilizer, a pH regulator, an antioxidant, a foam stabilizer, a diluent, a chelating agent, a propellant, and combinations thereof;
and, the humectant is selected from the group consisting of glycerol, polyethylene glycol 2000, polyethylene glycol 4000, polyethylene glycol 6000, and combinations thereof;
the solubilizer is selected from the group consisting of Tween 40, Tween 60, Tween 80, sodium dodecyl sulfate, docusate sodium, and combinations thereof;
the pH regulator is selected from the group consisting of lactic acid, hydrochloric acid, sodium hydroxide, acetic acid, and combinations thereof;
the antioxidant is selected from the group consisting of dibutyl hydroxytoluene (BHT), butyl hydroxyanisole (BHA), vitamin E, and combinations thereof;
the foam stabilizer is selected from the group consisting of cetyl alcohol, stearyl alcohol, and combinations thereof;
the diluent is selected from the group consisting of purified water, anhydrous ethanol, n-propanol, and combinations thereof;
the chelating agent is selected from the group consisting of lactic acid, anhydrous citric acid, acetic acid, malic acid, maleic acid, and combinations thereof;
the propellant is selected from the group consisting of propane, butane, isobutane, and combinations thereof.

7. The foam formulation of claim 6, wherein, calculated on a propellant-free basis, the mass percentage of the humectant in the foam formulation is 1-10 wt%; preferably 1-5 wt%; more preferably 2-3 wt%; more preferably 2-2.5 wt%;
calculated on a propellant-free basis, the mass percentage of the solubilizer in the foam formulation is 0.1-5.0 wt%; preferably 0.1-1.0 wt%; more preferably 0.1-0.5 wt%; more preferably 0.3-0.5 wt%;
calculated on a propellant-free basis, the mass percentage of the pH regulator in the foam formulation is 0.1-5.0 wt%; preferably 0.5-2.5 wt%; more preferably 1.0-2.0 wt%; more preferably 1.0-1.5 wt%;
calculated on a propellant-free basis, the mass percentage of the antioxidant in the foam formulation is 0.01-1 wt%; preferably 0.05-0.5 wt%; more preferably 0.1-0.2 wt%;
calculated on a propellant-free basis, the mass percentage of the foam stabilizer in the foam formulation is 0.5-10 wt%; preferably 0.5-5 wt%; more preferably 1.5-2.5 wt%; more preferably 1.5-2.0 wt%;
calculated on a propellant-free basis, the mass percentage of the chelating agent in the foam formulation is 0.1-5.0 wt%; preferably 0.1-1.0 wt%; more preferably 0.1-0.5 wt%; more preferably 0.1-0.3 wt%; more preferably 0.1-0.2 wt%;
calculated on a propellant-free basis, the mass percentage of the propellant in the foam formulation is 1.5-8.5 wt%; preferably 2.5-7.5 wt%; more preferably 3.5-6.5 wt%; more preferably 4.5-5.5 wt%.

8. The foam formulation of claim 6, wherein the foam formulation further comprises: glycerin, Tween 60, lactic acid, dibutyl hydroxytoluene, cetyl alcohol, stearyl alcohol, anhydrous citric acid, anhydrous ethanol, purified water, and a propellant.

9. A liniment formulation for treating alopecia, comprising the following active components on the basis of the mass/volume percentage (w/v):
(a) 5% minoxidil; and
(b) 0.07-0.095% finasteride;
preferably, comprising the following active components on the basis of the mass/volume percentage (w/v):
(a) 5% minoxidil; and
(b) 0.07-0.09% finasteride;
preferably, comprising the following active components on the basis of the mass/volume percentage (w/v):
(a) 5% minoxidil; and
(b) 0.07-0.085% finasteride;
preferably, comprising the following active components on the basis of the mass/volume percentage (w/v):
(a) 5% minoxidil; and
(b) 0.07-0.08% finasteride;
preferably, comprising the following active components on the basis of the mass/volume percentage (w/v):
(a) 5% minoxidil; and
(b) 0.07-0.075% finasteride;
more preferably, comprising the following active components on the basis of the mass/volume percentage (w/v):
(a) 5% minoxidil; and
(b) 0.075% finasteride.

10. The liniment formulation of claim 9, wherein the liniment formulation further comprises a penetration enhancer, a cosolvent, a solvent, aor combinations thereof;
and, the penetration enhancer is selected from the group consisting of propylene glycol, laurocapram, cyclohexanol, decyl methyl sulfoxide, and combinations thereof;
the cosolvent is selected from the group consisting of anhydrous ethanol, glycerol, isopropanol, polyethylene glycol, liquid paraffin, and combinations thereof.

11. The liniment formulation of claim 10, wherein the volume percentage (v/v) of the penetration enhancer in the liniment formulation is 35%-60% (v/v), preferably 45% -55% (v/v), and more preferably 50% -55% (v/v);
the volume percentage (v/v) of the cosolvent in the ointment is 15%-45% (v/v), preferably 20%-40% (v/v), and more preferably 25% -35% (v/v).

12. The liniment formulation of claim 10, comprising propylene glycol, anhydrous ethanol, and purified water.

13. Use of the formulation or composition of claim 1, the foam formulation of claim 5, or the liniment formulation of claim 9 in preparing a pharmaceutical for treating alopecia;
wherein, the treating comprises promoting hair growth and preventing further hair loss.

14. The use of claim 13, wherein the alopecia is male androgenic alopecia or female postmenopausal alopecia.

15. A method for preparing the foam formulation of claim 5, comprising the following steps:
(s1) taking the following active components as raw materials according to the mass percentage for subsequent use:
(a) 5% minoxidil; and
(b) 0.07-0.095% finasteride;
(s2) mixing the active components with a pharmaceutically acceptable carrier, dissolving, filtering, filling, and sealing; and
(s3) injecting a propellant into a sealed solution to prepare the foam formulation.

16. The method of claim 15, further comprising the following substeps in step (s2):
(s2a) preparing an aqueous phase: dissolving a chelating agent in purified water;
(s2b) preparing an alcohol phase: dissolving finasteride in a small amount of anhydrous ethanol, then adding a humectant, a solubilizer, a pH regulator, finasteride solution, an antioxidant, and a foam stabilizer into anhydrous ethanol for dissolution, and then adding the minoxidil, stirring to disperse; and
(s2c) mixing the aqueous phase(s2a) with the alcohol phase(s2b), stirring until the raw materials are completely dissolved, filtering, filling and sealing.

17. The method of claim 15, further comprising the following substeps in step (s2):
(s2a) preparing an aqueous phase: dissolving anhydrous citric acid in purified water;
(s2b) preparing an alcohol phase: dissolving the finasteride in a small amount of anhydrous ethanol, then adding glycerol, Tween 60, lactic acid, finasteride ethanol solution, dibutyl hydroxytoluene, cetyl alcohol, and stearyl alcohol to anhydrous ethanol for dissolution, and then adding the minoxidil, stirring to disperse; and
(s2c) mixing the aqueous phase (s2a) with the alcohol phase(s2b), stirring until the minoxidil is completely dissolved, filtering, filling and sealing.

18. A method for preparing the liniment formulation of claim 9, comprising the following steps:
(s1) taking the following active component raw materials according to the mass/volume percentage for subsequent use;
(a) 5% minoxidil; and
(b) 0.07-0.095% finasteride;
(s2) uniformly mixing the active component and a pharmaceutically acceptable carrier, adjusting to final volume, and filtering; and
(s3) filling a filtered solution to obtain the liniment formulation.

19. The method of claim 18, further comprising the following substeps in step (s2):
(s2a) dissolving finasteride in a small amount of cosolvent;
(s2b) uniformly mixing a penetration enhancer, a cosolvent, and a first portion of solvent, then adding minoxidil and finasteride solution and stirring to dissolve; and
(s2c) adjusting the volume of a solution obtained after dissolution with the remaining solvent to final volume and filtering.

20. The method of claim 18, further comprising the following substeps in step (s2):
(s2a) dissolving finasteride in a small amount of anhydrous ethanol;
(s2b) uniformly mixing the propylene glycol, anhydrous ethanol, and a first portion of purified water, adding minoxidil and finasteride ethanol solution, stirring to dissolve; and
(s2c) diluting the solution obtained after dissolution with remaining purified water and filtering.
